# EUROPEAN PATENT SPECIFICATION

(11) **EP 1 670 440 B1**
(45) Date of publication and mention of the grant of the patent: **30.04.2014**
(21) Application number: 04762858.1
(22) Date of filing: 21.09.2004
(51) Int. Cl.: A61K 31/56, A61K 31/57, A61K 9/20

(54) **HRT FORMULATIONS**
HRT FORMULIERUNGEN
FORMULATIONS HRT

(30) Priority: 29.09.2003 DK 200301408; 09.10.2003 US 509962 P; 10.12.2003 DK 200301828; 13.01.2004 US 536121 P
(43) Date of publication of application: 21.06.2006
(60) Divisional application: 10178130.0
(73) Proprietor: Novo Nordisk Femcare AG, 8050 Zürich (CH)
(72) Inventor: EDWARDS, WILLIAM, Martin, Medstead, Hants GU34 5JF (GB); SHALMI, Michael, 2900 Hellerup (DK); JENSEN, MOLLER, Bente, 2630 Taastrup (DK)
(74) Representative: Noergaard, Torsten
(86) International application number: PCT/DK2004/000638
(87) International publication number: WO 2005/030175

(56) References cited:
- US-A- 4 826 831
- "VIDAL" 1997, EDITIONS DU VIDAL , PARIS , XP002316912 page 1699 - page 1700
- "VIDAL" 1997, EDITIONS DU VIDAL , PARIS , XP002316913 page 903 - page 904
- EDITIO CANTOR VERLAG: "ROTE LISTE" 2002, ROTE LISTE SERVICE GMBH , FRANKFURT/MAIN, GERMANY , XP002316914 pages 76-087
- EDITIO CANTOR VERLAG: "ROTE LISTE 2002" 2002, ROTE LISTE SERVICE GMBH , FRANKFURT/MAIN , XP002316915 pages 76-110

## Description

### Field of this Invention

The present invention relates to novel pharmaceutical formulations containing surprisingly low concentrations of estradiol and norethindrone acetate (hereinafter designated NETA). These formulations are to be used for the treatment of hormone placement therapy (hereinafter designated HRT).

### Background of this Invention

A HRT formulation designated Kliogest® was marketed in the beginning of the 80'ies. Each Kliogest tablet contains, as active ingredients, 2 mg of estradiol and 1 mg of NETA. Also, at that time, it was suggested that, at least to some patients, half the dosage, i.e. a tablet containing 1 mg of estradiol and 0.5 mg of NETA, was the optimal dosage. The last mentioned product has been marketed under the name Activelle®. The daily administration is one Kliogest tablet. The administration takes place for a long period of time, usually months or even years.

Examples of documents defining the general state of the art are the following patents and patent applications: US 4,826,831 A; WO 02/055086 A2; and US 2002/193358 A.

It is the object of this invention to find a HRT formulation that is extremely satisfactory to the patients.

It is the object of this invention to find a HRT formulation giving a satisfactory and convenient bleeding profile.

### Summary of this Invention

It has now, surprisingly, been found that the object can be obtained when 0.5 mg of estradiol and 0.1 mg of NETA is administered daily.

In the formulations of this invention, estradiol may be present as such or it may be present as the corresponding amount of a hydrate thereof, for example, the corresponding amount of the hemihydrate (estradiol hemihydrate).

Furthermore, in the formulations of this invention, NETA may be present as such or it may be present as the corresponding amount of norethindrone (hereinafter designated NET) or the corresponding amount of a salt or ester of NET.

In a preferred embodiment, the unit dosage form is a pellet, tablet or capsule having a total weight (excluding capsule or coating) of between about 25 and 125 mg.

In some embodiments, the formulation comprises:

| | |
|---|---|
| Estradiol hemihydrate | about 0.5 mg |
| Norethisterone acetate | about 0.1 mg |
| Lactose monohydrate | about 37 mg |
| Maize starch | about 37 mg |
| Hydroxypropyl cellulose or hydroxypropyl methyl cellulose | about 3 mg |
| Talc | about 0.8 mg |
| Magnesium stearate | about 0.4 mg |

In another aspect, the invention provides methods for treating a progestogen-responsive syndrome, which are carried out by administering to a patient in need of such treatment an effective amount for treating the syndrome of a formulation of the invention.

### Detailed Description of the Invention

The present invention relates to formulations of estradiol-containing and NETA-containing medications. Such formulations can, for example, be administered orally, vaginally, or transdermally.

The formulations of this invention can be used analogously to the use of similar products such as Kliogest and Activelle.

Examples of pharmaceutically acceptable salts, including, without limitation, organic acid addition salts formed with acids which form a physiological acceptable anion, for example, tosylate, methanesulfonate, acetate, citrate, malonate, tartarate, succinate, benzoate, ascorbate, α-ketoglutarate, and α-glycerophosphate. Suitable inorganic salts may also be formed, including, without limitation, hydrochloride, sulfate, nitrate, bicarbonate, and carbonate salts.

In practicing the present invention, an estradiol-containing and NETA-containing formulation is prepared by contacting estradiola and NETA with a cellulosic binder. The binder may comprise, without limitation, one or more of methylcellulose, sodium carboxymethylcellulose (Tylose™), ethylcellulose (Ethocel™), hydroxypropyl methyl cellulose (HPMC) (Shin-Etsu Chemical Co., Ltd.), and hydroxypropyl cellulose (Klucel™). For tablet formulations, binders impart sufficient cohesion to the powders to permit normal processing such as sizing, lubrication, compression, film coating, and packaging, but still permit the tablet to disintegrate and the formulation to dissolve upon ingestion. In the present invention, the cellulosic binders also impart enhanced stability and/or recoverability to the progestogen active ingredient. Typically, the formulations of the invention comprise one or more cellulosic binders in the range of about 0.5% to about 25%, such as, e.g., about 0.75% to about 15% or about 1.5% to about 10%, of the total weight of the unit dosage form.

In one embodiment of the invention, the binder is Klucel™, and the unit dosage form is a tablet containing 0.1 mg NETA and 3.2 mg Klucel™ (total tablet weight = 80-85 mg).

The formulations of the invention may optionally comprise one or more diluents. Suitable diluents include, without limitation, either individually or in combination, lactose USP; lactose USP, anhydrous; lactose USP, spray dried; starch USP; directly compressible starch; mannitol USP; sorbitol; dextrose monohydrate; microcrystalline cellulose NF; dibasic calcium phosphate dihydrate NF; sucrose-based diluents; confectioner's sugar; monobasic calcium sulfate monohydrate; calcium sulfate dihydrate NF; calcium lactate trihydrate granular NF; dextrates NF (e.g., Emdex^{™}); Celutab^{™}; dextrose (e.g., Cerelose^{™}); inositol; hydrolyzed cereal solids such as the Maltrons^{™} and Mor-Rex^{™}; amylose; Rexcel^{™}; calcium carbonate; glycine; bentonite; and the like. The formulations typically comprise one or more diluents in the range of about 5% to about 99%, preferably about 25% to about 90%, and more preferably about 40% to about 80%, of the total weight of the formulation.

The formulations of the invention may optionally comprise one or more disintegrants, particularly for tablet formulations. Suitable disintegrants include, without limitation, either individually or in combination, starches; sodium starch glycolate; clays (such as Veegum^{™}HV); alginates; pregelatinized corn starches (such as National^{™} 1551 and National^{™} 1550); and gums (such as agar, guar, locust bean, Karaya^{™}, pectin, and tragacanth). Disintegrants can be added at any suitable step during the preparation of the pharmaceutical formulation, particularly prior to granulation or during the lubrication step prior to compression. Preferably, the present pharmaceutical formulations comprise one or more disintegrants in the range of about 5% to about 99%, preferably about 25% to about 90%, and more preferably about 40% to about 80%, of the total weight of the formulation.

The formulations of the invention optionally comprise one or more lubricants and/or glidants as a carrier material. Suitable lubricants and/or glidants include, without limitation, either individually or in combination, such lubricants and/or glidants as glyceryl behenate (Compritol^{™} 888); metalllic stearates (e.g., magnesium, calcium and sodium stearates); stearic acid; hydrogenated vegetable oils (e.g., Sterotex^{™}); talc; waxes; Stearowet^{™}; boric acid; sodium benzoate and sodium acetate; sodium chloride; DL-Leucine; polyethylene glycols (e.g., Carbowax^{™} 4000 and Carbowax^{™} 6000); sodium oleate; sodium benzoate; sodium acetate; sodium lauryl sulfate; sodium stearyl fumarate (Pruv.TM.); and magnesium lauryl sulfate. The present pharmaceutical formulations comprise one or more lubricants at about 0.1 % to about 10%, preferably about 0.2% to about 8%, and more preferably about 0.25% to about 5%, of the total weight of the formulation. Magnesium stearate is a preferred lubricant used to reduce friction between the equipment and granulation during compression.

Talc is a preferred anti-adherent or glidant agent used to reduce sticking to equipment surfaces and also to reduce static in the blend. The formulations preferably comprise talc at about 0.1 % to about 10%, more preferably about 0.25% to about 5%, and still more preferably about 0.5% to about 2%, of the total weight of the formulation.

The formulations of the invention optionally comprise one or more wetting agents, particularly for tablet formulations. Suitable wetting agents include, either individually or in combination, such wetting agents as oleic acid; glyceryl monostearate; sorbitan monooleate; sorbitan monolaurate; triethanolamine oleate; polyoxyethylene sorbitan mono-oleate; polyoxyethylene sorbitan monolaurate; sodium oleate; and sodium lauryl sulfate. Wetting agents that are anionic surfactants are preferred. The formulations of the invention typically comprise one or more wetting agents present at about 0.1 % to about 15%, preferably about 0.25% to about 10%, and more preferably about 0.5% to about 5%, of the total weight of the formulation.

Other carrier materials (such as colorants, flavors and sweeteners) and modes of administration are known in the pharmaceutical art and can be used in the preparation of the formulations of the present invention.

### Dosage forms and coatings

A dosage form, as used herein, refers to a formulation that is ready for administration to a subject. In practicing the present invention, the formulation may be formulated as tablets, pills, hard or soft capsules, lozenges, cachets, dispensable powders, granules, beadlets, pellets, suspensions, elixirs, and the like. The invention also encompasses multilayered tablets wherein a given layer may represent a different drug, as well as powders, pellets and granules that are encapsulated. The powders, pellets, and granules may be coated with a suitable polymer or a conventional coating material to achieve, for example, greater stability in the gastrointestinal tract, to achieve the desired rate of release, or to achieve a product with improved stability. Moreover, the capsule comprising the powder, pellets or granules may be further coated. The tablet or the caplet may also be scored to facilitate division of dosing. Alternatively, the dosage forms of the present invention may be unit dosage forms wherein the dosage form is intended to deliver one therapeutic dose per administration.

Preferably, the formulation is formulated into a discrete dosage unit containing a predetermined amount of the active ingredients, such as tablets or capsules. Unit dosage tablets or capsules are preferred.

Coatings typically comprise one or more compounds that form a film, as well as one or more plasticizers.

Film-forming compounds include, without limitation, cellulosic compounds such as, e.g., hydroxypropyl methyl cellulose, ethylcellulose, methacrylates, cellulose acetate phthalates, polyvinyl acetate phthalates, waxes, and silicone elastomers.

Plasticizers include, without limitation, glycerin, glycerol triacetate, propylene glycol, polyethylene glycosl, triacetin, triethyl citrate, acetylated monoglycerides MACROGOL 6,000, and the like.

In one aspect, the invention provides progestogen-containing tablets coated with 1.5-5% w/w of a cellulosic film-forming compound relative to the total weight of the tablet, such as, e.g., 1.5%, 2%, 2.5%, 3%, 3.5%, 4%, 4.5, and 5% w/w.

In one embodiment, the formulation is a tablet containing 0.1 mg NETA and 3.2 mg Klucel™ (total tablet weight = 80-85 mg) and coated with 3% methylhydroxypropylcellulose E3 (total weight about 2.3 mg/tablet).

The formulations of the invention may be manufactured using conventional means of granulation and tabletting, including, without limitation, wet granulation and tabletting followed by film-coating or direct compression followed by film-coating.

Typically, the tablet process comprises the steps of preparing granules suitable for tableting by blending a mixture comprising the medicament, a binder, and optionally, a disintegrant and filler; adding a predetermined amount of water or granulation fluid to form a wet mass blend; sizing the wet mass blend into granules to aid drying; drying the wet granules to remove excess moisture; sizing the dried granules into granules suitable for tableting, and adding lubricant, one or more fillers, one or more dry binders, optionally a disintegrant, and other excipients necessary for tableting the granules; and a film-coating step.

Methods for preparing the formulations of the invention are well-known in the art and can be found, e.g., in Remington, 19^{th} Edition, Vol. II, Chapter 92, (Mack Publishing Company, Easton PA, 1995).

### Other active ingredients

In addition to progestogen, the formulations of the invention may comprise one or more additional active ingredients, including, without limitation, a second progestogen product, an androgenic agent, an androgen agonist, a progestogen agonist, an estrogen antagonist, or another hormone product.

In one embodiment, the formulation is a tablet containing 0.5 mg estradiol in addition to 0.1 mg NETA.
The formulation of this invention, particularly a dry formulation such as, e.g., a tablet, can be prepared by contacting estradiol and NETA with a cellulosic binder such that the cellulosic binder comprises between about 0.5% to about 25%, such as, e.g., about 0.75% to about 15% or about 1% to about 10%, of the total weight of the formulation. Non-limiting examples of suitable cellulosic binders include methylcellulose, sodium carboxymethylcellulose (Tylose™), ethylcellulose (Ethocel™), hydroxypropyl methyl cellulose (HPMC) (Shin-Etsu Chemical Co., Ltd.), and hydroxypropyl cellulose (Klucel™). However, it will be understood that any cellulosic binder may be used that imparts enhanced stability and/or recoverability to the formulation.

In another aspect, the formulation of this invention, particularly a dry formulation such as, e.g., a tablet, can be prepared by coating the estrogen-containing and NETA-containing dosage form with 0.5-5% w/w of a cellulosic film-forming compound relative to the total weight of the tablet, such as, e.g., 1.5%, 2%, 2.5%, 3%, 3.5%, 4%, 4.5, and 5% w/w.

### Methods of treatment

The present invention also encompasses methods for treatment of progestogen-responsive syndromes, such as, e.g., in hormone replacement therapy. Also encompassed are other modes of progestogen administration such as, e.g., for contraception and treatment of osteoporosis. The methods are carried out by administering the formulation described above to a subject in need of a progestogen (or, a combination of a progestogen and another active ingredient, such as, e.g., an estrogen). Progestogen-responsive syndromes include, without limitation, infertility related to non-receptive uterus, premenstrual tension, ovulation, primary dysmenorrhea and endometriosis, habitual abortion, respiratory depression in the Pickwickian syndrome, secondary amenorrhea, dysfunctional uterine bleeding, preeclampsia and toxemia of pregnancy, sexual infantilism, and post-menopausal symptoms.

The following examples are intended as non-limiting illustrations of the present invention.

### Example 1: Enhanced stability of NETA in a low-dose formulation

The purpose of this study was to compare the effect of (i) different binders and (ii) different coatings on stability of NETA is a low-dose formulation.

The following table illustrates the test formulations:

| **Ingredients** | **Quantity (mg/tablet)** | **Function** | **Commercial Source** |
|---|---|---|---|
| **Active ingredients** | | | |
| Estradiol hemihydrate | 0.517 | Active substance | Schering AG, Germany / Diosynth B.V., Holland |
| Norethisterone Acetate | 0.100 | Active substance | Schering/Diosynth |

| **Other ingredients** | | | |
|---|---|---|---|
| Lactose monohydrate | 37.5 | Filler | DMV International, Holland |
| Maize starch | 37.5 | Disintegrant / filler | Cerestar Scandinavia |
| Binder | 3.20 | Binder | |
| Talc | 0.800 | Glidant / lubricant | Luzenac, Italy |
| Magnesium stearate | 0.400 | Lubricant | Acros Chemicals |

Two different binders were tested: Polyvidon™ VA 64 (polyvinylpyrollidone, BASF, Germany); and Klucel™ EF (hydroxypropyl cellulose, Aqualon, USA).

Tabletting and coating: Tablets containing each of the three cellulosic binders were formed by fluid-bed granulation, compression on rotary press, film-coating in a coating pan using an air atomizing spray system. For film coating, either 1% or 3% Methocel E3 or E5 was used. The final tablets contained the following coat components:

**1% film coat:**

| | |
|---|---|
| **Film-coating: (theoretical quantity mg/tablet)** | 0.864 |
| Filmformer | 0.821 |
| Glycerol triacetate | 0.043 |

**3% film coat:**

| | |
|---|---|
| **Film-coating: (theoretical quantity mg/tablet)** | 2.400 |
| Filmformer | 2.280 |
| Glycerol triacetate | 0.120 |

Packaging: Following coating, each tablet batch was packed in a calendar dial pack ("Dispenser").

Many variations of the present invention will suggest themselves to those skilled in the art in light of the above detailed description. Such obvious variations are within the full intended scope of the appended claims.

Herein the word "comprise" is to be interpreted broadly meaning "include", "contain" or "comprehend" (vide, for example, Guidelines for Examination in the European Patent Office, Part C, Chapter III, 4.13).

## Claims

1. A pharmaceutical formulation containing low concentrations of estradiol and norethindrone acetate (NETA) comprising 0.5 mg of estradiol, optionally as a hydrate thereof, and 0.1 mg of NETA or the corresponding amount of norethindrone (NET) or a corresponding amount of an ester or a salt of NET.

2. The formulation according to the preceding claim, wherein the content of NETA is 0.1 mg.

3. The formulation according to any one of the preceding claim, which formulation is to be administered daily.

4. The formulation according to any one of the preceding claims which is a tablet, pill, hard or soft capsule, lozenge, cachet, dispensable powder, granule, beadlet, pellet, suspension, or elixir, preferably a tablet, pill, hard or soft capsule.

5. The formulation according to any one of the preceding claims, which has a loss of mass w/w of not more than 15%, preferably of not more than 10 %, when tested according to European Pharmacopoeia 4^{th} edition 2002, item 2.2.32, test C.

6. The formulation according to any one of the preceding claims, which by the method described in European Pharmacopoeia 4^{th} edition 2002, item 2.9.1, test A, disintegrates within 2 hours, preferably 1 hour, more preferred 30 minutes, most preferred 15 minutes.

7. The formulation according to any one of the preceding claims, containing a cellulosic binder selected from the group consisting of methylcellulose, sodium carboxyethyl-cellulose, ethylcellulose, hydroxypropyl methyl cellulose and hydroxypropyl cellulose.

8. The formulation according to the preceding claim, wherein said cellulosic binder is selected from the group consisting of Tylose™, Ethocel™ and Klucel™.

9. The formulation according to any one of the preceding claims, wherein the content of cellulosic binder is in the range from 2 to 10% w/w relative to the total formulation.

10. The formulation according to the preceding claim, wherein said cellulosic binder is hydroxypropyl cellulose.

11. The formulation according to the preceding claim, wherein said hydroxypropyl cellulose is Klucel™.

12. The formulation according to the preceding claim, wherein estradiol is present as a hydrate, preferably a hemihydrate.

13. The formulation according to any one of the preceding claims, containing lactose in an amount in the range from 30 to 45 mg, optionally as lactose monohydrate.

14. The formulation according to any one of the preceding claims, containing maize starch in an amount in the range from 30 to 45 mg.

15. The formulation according to any one of the preceding claims, containing hydroxypropylcellulose in an amount in the range from 2 to 10 mg.

16. The formulation according to any one of the preceding claims, containing talc in an amount in the range from 0.5 to 2 mg.

17. The formulation according to any one of the preceding claims, containing magnesium stearate in an amount in the range from 0.1 to 1 mg.

18. The formulation according to any one of the preceding claims, containing hydroxypropylmethyl cellulose as film coater in an amount in the range from 0.5 to 3 mg.

19. The formulation according to any one of the preceding claims, containing glycerol triacetate in an amount in the range from 0.05 to 0.2 mg.

20. The formulation according to any one of the preceding claims, comprising an amount in the range from 2.5 to 4 mg of a cellulosic binder, wherein said cellulosic binder is selected from the group consisting of methylcellulose, sodium carboxymethylcellulose, ethylcellulose, hydroxypropyl methyl cellulose, and hydroxypropyl cellulose.

21. The formulation according to any one of the preceding claims, comprising 40 mg of lactose monohydrate, 40 mg of maize starch, 3 mg of hydroxypropyl cellulose or hydroxypropyl methyl cellulose, 0.8 mg of talc, and 0.4 mg of magnesium stearate.

22. The formulation according to any one of the preceding claims, further comprising a cellulosic coating.

23. The formulation according to any one of the preceding claims, wherein said cellulosic coating is hydropropyl methyl cellulose.

24. The formulation according to any one of the preceding claims, wherein said coating is present in an amount in the range from 0.5 to 5% w/w relative to the total formulation.

25. The formulation according to the preceding claim, wherein said coating is present in an amount in the range from 2 to 4% w/w relative to the total formulation.

26. The formulation according to any one of the preceding claims, wherein said coating is present in an amount in the range from 2.5 to 3.5% w/w relative to the total formulation.

27. The formulation according to any one of the preceding claims, wherein the total amount of said coating is in the range from 2 to 3 mg per unit dosage.

28. A formulation according to any one of the preceding claims for treating a progestogen-responsive syndrome.

## Patentansprüche

1. Pharmazeutische Formulierung, enthaltend geringe Konzentrationen an Estradiol und Norethindronacetat (NETA), umfassend 0,5 mg Estradiol, wahlweise als Hydrat davon, und 0,1 mg NETA oder die entsprechende Menge an Norethindron (NET) oder eine entsprechende Menge eines Esters oder Salzes von NET.

2. Formulierung nach dem vorangehenden Anspruch, wobei der Gehalt an NETA 0,1 mg beträgt.

3. Formulierung nach einem der vorangehenden Ansprüche, wobei die Formulierung täglich zu verabreichen ist.

4. Formulierung nach einem der vorangehenden Ansprüche, bei welcher es sich um eine Tablette, eine Pille, eine Hart- oder Weichkapsel, eine Pastille, ein Cachet, ein dosierbares Pulver, ein Granulat, ein Kügelchen, ein Pellet, eine Suspension oder ein Elixier, vorzugsweise eine Tablette, eine Pille, eine Hart- oder Weichkapsel handelt.

5. Formulierung nach einem der vorangehenden Ansprüche, die beim Testen gemäß der Europäischen Pharmakopöe, 4. Ausgabe 2002, Punkt 2.2.32, Test C, einen Massenverlust G/G von nicht mehr als 15%, vorzugsweise nicht mehr als 10% aufweist.

6. Formulierung nach einem der vorangehenden Ansprüche, die durch das in der Europäischen Pharmakopöe, 4. Ausgabe 2002, Punkt 2.9.1, Test A, beschriebene Verfahren innerhalb von 2 Stunden, vorzugsweise 1 Stunde, stärker bevorzugt 30 Minuten, besonders bevorzugt 15 Minuten abgebaut wird.

7. Formulierung nach einem der vorangehenden Ansprüche, enthaltend ein Cellulosebindemittel, ausgewählt aus der Gruppe, bestehend aus Methylcellulose, Natriumcarboxyethylcellulose, Ethylcellulose, Hydroxypropylmethylcellulose und Hydroxypropylcellulose.

8. Formulierung nach dem vorangehenden Anspruch, wobei das Cellulosebindemittel ausgewählt ist aus der Gruppe, bestehend aus Tylose™, Ethocel™ und Klucel™.

9. Formulierung nach einem der vorangehenden Ansprüche, wobei der Gehalt an Cellulosebindemittel im Bereich von 2 bis 10 Gew.- % in Bezug auf die Formulierung liegt.

10. Formulierung nach dem vorangehenden Anspruch, wobei es sich bei dem Cellulosebindemittel um Hydroxypropylcellulose handelt.

11. Formulierung nach dem vorangehenden Anspruch, wobei es sich bei der Hydroxypropylcellulose um Klucel™ handelt.

12. Formulierung nach dem vorangehenden Anspruch, wobei Estradiol als Hydrat, vorzugsweise Hemihydrat vorliegt.

13. Formulierung nach einem der vorangehenden Ansprüche, die Lactose in einer Menge im Bereich von 30 bis 45 mg, wahlweise als Lactosemonohydrat enthält.

14. Formulierung nach einem der vorangehenden Ansprüche, die Maisstärke in einer Menge im Bereich von 30 bis 45 mg enthält.

15. Formulierung nach einem der vorangehenden Ansprüche, die Hydroxypropylcellulose in einer Menge im Bereich von 2 bis 10 mg enthält.

16. Formulierung nach einem der vorangehenden Ansprüche, die Talkum in einer Menge im Bereich von 0,5 bis 2 mg enthält.

17. Formulierung nach einem der vorangehenden Ansprüche, die Magnesiumstearat in einer Menge im Bereich von 0,1 bis 1 mg enthält.

18. Formulierung nach einem der vorangehenden Ansprüche, die Hydroxypropylmethylcellulose als Filmbeschichtungsmittel in einer Menge im Bereich von 0,5 bis 3 mg enthält.

19. Formulierung nach einem der vorangehenden Ansprüche, die Glycerintriacetat in einer Menge im Bereich von 0,05 bis 0,2 mg enthält.

20. Formulierung nach einem der vorangehenden Ansprüche, umfassend eine Menge im Bereich von 2,5 bis 4 mg eines Cellulosebindemittels, wobei das Cellulosebindemittel ausgewählt ist aus der Gruppe, bestehend aus Methylcellulose, Natriumcarboxymethylcellulose, Ethylcellulose, Hydroxypropylmethylcellulose und Hydroxypropylcellulose.

21. Formulierung nach einem der vorangehenden Ansprüche, umfassend 40 mg Lactosemonohydrat, 40 mg Maisstärke, 3 mg Hydroxypropylcellulose oder Hydroxypropylmethylcellulose, 0,8 mg Talkum und 0,4 mg Magnesiumstearat.

22. Formulierung nach einem der vorangehenden Ansprüche, des Weiteren umfassend eine Cellulosebeschichtung.

23. Formulierung nach einem der vorangehenden Ansprüche, wobei es sich bei der Cellulosebeschichtung um Hydroxypropylmethylcellulose handelt.

24. Formulierung nach einem der vorangehenden Ansprüche, wobei die Beschichtung in einer Menge im Bereich von 0,5 bis 5 Gew.-% in Bezug auf die gesamte Formulierung vorliegt.

25. Formulierung nach dem vorangehenden Anspruch, wobei die Beschichtung in einer Menge im Bereich von 2 bis 4 Gew.-% in Bezug auf die gesamte Formulierung vorliegt.

26. Formulierung nach einem der vorangehenden Ansprüche, wobei die Beschichtung in einer Menge im Bereich von 2,5 bis 3,5 Gew.- % in Bezug auf die gesamte Formulierung vorliegt.

27. Formulierung nach einem der vorangehenden Ansprüche, wobei die Gesamtmenge der Beschichtung im Bereich von 2 bis 3 mg pro Einheitsdosis vorliegt.

28. Formulierung nach einem der vorangehenden Ansprüche zum Behandeln eines auf Progesteron ansprechbaren Syndroms.

## Revendications

1. Formulation pharmaceutique contenant de faibles concentrations d'oestradiol et d'acétate de noréthindrone (NETA) comprenant 0,5 mg d'oestradiol, en option sous forme d'un hydrate de ce dernier, et 0,1 mg de NETA ou la quantité correspondante de noréthindrone (NET) ou une quantité correspondante d'un ester ou d'un sel de NET.

2. Formulation selon la revendication précédente, dont la teneur en NETA est de 0,1 mg.

3. Formulation selon l'une quelconque des revendications précédentes, laquelle formulation doit être administrée quotidiennement.

4. Formulation selon l'une quelconque des revendications précédentes, qui est un comprimé, une pilule, une gélule dure ou une capsule molle, une pastille, un cachet, une poudre dispersible, un granulé, des microbilles, un pellet, une suspension ou un élixir, de préférence un comprimé, une pilule, une gélule dure ou une capsule molle.

5. Formulation selon l'une quelconque des revendications précédentes, qui présente une perte de masse p/p non supérieure à 15 %, de préférence non supérieure à 10 %, l'essai étant effectuée selon la Pharmacopée Européenne, 4ème édition 2002, poste 2.2.32, test C.

6. Formulation selon l'une quelconque des revendications précédentes, qui par la méthode décrite dans la Pharmacopée Européenne, 4ème édition 2002, poste 2.9.1, test A, se désintègre dans les 2 heures, de préférence en 1 heure, d'une manière plus préférée en 30 minutes, d'une manière spécialement préférée en 15 minutes.

7. Formulation selon l'une quelconque des revendications précédentes, contenant un liant cellulosique choisi dans le groupe consistant en la méthylcellulose, la carboxyéthylcellulose sodique, l'éthylcellulose, l'hydropropylméthylcellulose et l'hydroxypropylcellulose.

8. Formulation selon la revendication précédente, dans laquelle ledit liant cellulosique est choisi dans le groupe consistant en le Tylose^{™}, l'Ethocel^{™} et le Klucel^{™}.

9. Formulation selon l'une quelconque des revendications précédentes, dont la teneur en le liant cellulosique est comprise dans la plage de 2 à 10 % p/p par rapport à la formulation totale.

10. Formulation selon la revendication précédente, dans laquelle ledit liant cellulosique est l'hydroxypropylcellulose.

11. Formulation selon la revendication précédente, dans laquelle ladite hydroxypropylcellulose est le Klucel^{™}.

12. Formulation selon la revendication précédente, dans laquelle l'oestradiol est présent sous forme d'un hydrate, de préférence d'un hémihydrate.

13. Formulation selon l'une quelconque des revendications précédentes, contenant du lactose en une quantité comprise dans la plage de 30 à 45 mg, en option sous forme de lactose monohydraté.

14. Formulation selon l'une quelconque des revendications précédentes, contenant de l'amidon de maïs en une quantité comprise dans la plage de 30 à 45 mg.

15. Formulation selon l'une quelconque des revendications précédentes, contenant de l'hydroxypropylcellulose en une quantité comprise dans la plage de 2 à 10 mg.

16. Formulation selon l'une quelconque des revendications précédentes, contenant du talc en une quantité comprise dans la plage de 0,5 à 2 mg.

17. Formulation selon l'une quelconque des revendications précédentes, contenant du stéarate de magnésium en une quantité comprise dans la plage de 0,1 à 1 mg.

18. Formulation selon l'une quelconque des revendications précédentes, contenant de l'hydroxypropylméthylcellulose en tant que pelliculage en une quantité comprise dans la plage de 0,5 à 3 mg.

19. Formulation selon l'une quelconque des revendications précédentes, contenant du triacétate de glycérol en une quantité comprise dans la plage de 0,05 à 0,2 mg.

20. Formulation selon l'une quelconque des revendications précédentes, comprenant une quantité comprise dans la plage de 2,5 à 4 mg d'un liant cellulosique, ledit liant cellulosique étant choisi dans le groupe consistant en la méthylcellulose, la carboxyméthylcellulose sodique, l'éthylcellulose, l'hydroxypropylméthylcellulose et l'hydroxypropylcellulose.

21. Formulation selon l'une quelconque des revendications précédentes, comprenant 40 mg de lactose monohydraté, 40 mg d'amidon de maïs, 3 mg d'hydroxypropylcellulose ou d'hydroxypropylméthylcellulose, 0,8 mg de talc et 0,4 mg de stéarate de magnésium.

22. Formulation selon l'une quelconque des revendications précédentes, qui comprend en outre un enrobage cellulosique.

23. Formulation selon l'une quelconque des revendications précédentes, dans laquelle ledit enrobage cellulosique est l'hydroxypropylméthylcellulose.

24. Formulation selon l'une quelconque des revendications précédentes, dans laquelle ledit enrobage est présent en une quantité comprise dans la plage de 0,5 à 5 % p/p par rapport à la formulation totale.

25. Formulation selon la revendication précédente, dans laquelle ledit enrobage est présent en une quantité comprise dans la plage de 2 à 4 % p/p par rapport à la formulation totale.

26. Formulation selon l'une quelconque des revendications précédentes, dans laquelle ledit enrobage est présent en une quantité comprise dans la plage de 2,5 à 3,5 % p/p par rapport à la formulation totale.

27. Formulation selon l'une quelconque des revendications précédentes, dans laquelle la quantité totale dudit enrobage est comprise dans la plage de 2 à 3 mg par unité posologique.

28. Formulation selon l'une quelconque des revendications précédentes pour le traitement d'un syndrome de sensibilité aux progestagènes.
